# EUROPEAN PATENT SPECIFICATION

(11) **EP 2 421 592 B1**
(45) Date of publication and mention of the grant of the patent: **29.06.2016**
(21) Application number: 10767795.7
(22) Date of filing: 22.04.2010
(51) Int. Cl.: A61M 25/10

(54) **BALLOON CATHETER**
BALLONKATHETER
CATHÉTER À BALLONNET

(30) Priority: 22.04.2009 US 428077
(43) Date of publication of application: 29.02.2012
(73) Proprietor: Kurth, Paul, Teton Village, Wyoming 83025 (US); Armour, Andrew, Swathmore, Pennsylvania 19081 (US); Pressure Products Medical Supplies Inc., Teton Village, Wyoming 83025 (US)
(72) Inventor: KURTH, Paul, Teton Village, Wyoming 83025 (US); ARMOUR, Andrew, Swathmore, Pennsylvania 19081 (US)
(74) Representative: Duxbury, Stephen
(86) International application number: PCT/US2010/032120
(87) International publication number: WO 2010/124135

(56) References cited:
- WO-A2-2008/024911
- US-A- 4 311 146
- US-A1- 2004 111 145
- US-A1- 2005 148 997
- US-A1- 2007 112 422
- US-A1- 2008 114 333

## Description

### Background of the Invention

### Field of the Invention

The invention relates to the field of cardiac balloon catheters and the methods of manufacture of the same,

### Description of the Prior Art

Cardiac balloon catheters are well known in the art, having been introduced by Swan and Ganz in the 1970's as balloon flotation catheters. Versions of these catheters are currently used for temporary vascular occlusion; ie Swan-Ganz and Pulmonary Angiography catheters by Edwards Lifesciences (Irvine, CA) and Corodyn Right Heart Catheters by B. Braun (Bethlehem, PA). These catheters are dual lumen catheters with a distal compliant balloon bonded to the distal end in fluid communication with one of the lumens, with the other lumen being used for injection of radiopaque dye for performing angiography of the pulmonary arteries. The limitations of these catheters when being used for coronary sinus venography in a patient with heart failure is that the balloon is inherently too small to occlude the coronary sinus vein, a larger balloon size will not fit through the standard size introducers, and the larger balloon sizes inflate at too high of pressures, risking dissection of the coronary sinus vein.

Occlusive balloon catheters for performing coronary sinus venography are delivered by a delivery catheter or introducer to the coronary ostium or inlet to the coronary sinus in the right atrium. The coronary sinus is a large vein into which blood returning from the heart muscles empties before flowing through the coronary ostium into the right atrium. Thus, the coronary sinus has a larger diameter or cross section than any of the several coronary branch vessels emptying into it. In order to inject contrast agent into the coronary vessels to then fluoroscopically visualize and perform a cardiac intervention of any kind, it is necessary to inflate a balloon in the sinus to block the flow of blood returning to the atrium. Conventional balloons have a limited ratio of the inflated-to-deflated balloon diameter. In conventional designs, the larger the inflated balloon diameter, the larger the deflated balloon diameter. However, due to the large average diameter of the coronary sinus and the restrictive diameter of the coronary sinus delivery catheters or introducers through which the deflated balloon must first pass, the largest inflated balloon diameter achievable is usually only effective for occluding small coronary sinus vessels resulting in less than ideal coronary sinus venograms. Even more troublesome is the tendency of balloons of conventional design to fail to deflate flatly after being inflated. The larger the balloon, the more likely it is that it will be difficult to deflate the balloon to a size which can be easily withdrawn into the delivery catheter for removal from the vascular system. In addition, the balloon shape of conventional designs exhibits a length longer than the balloon diameter. This results in the potential to occlude the coronary branch vessels that are located near the coronary sinus ostium when performing coronary sinus venography.

What is needed is some kind of a design for a balloon catheter that will always allow its easy insertion into the coronary sinus, its inflation to a diameter effective to block the coronary sinus at any location in the sinus, to limit the balloon inflation forces that might cause dissection of the vein, to have a balloon shape whereby the diameter is equal to or larger than the balloon length, and the ability to thereafter deflate the balloon and fold it flatly to allow easy withdrawal into the delivery catheter or introducer regardless of its inflated diameter.

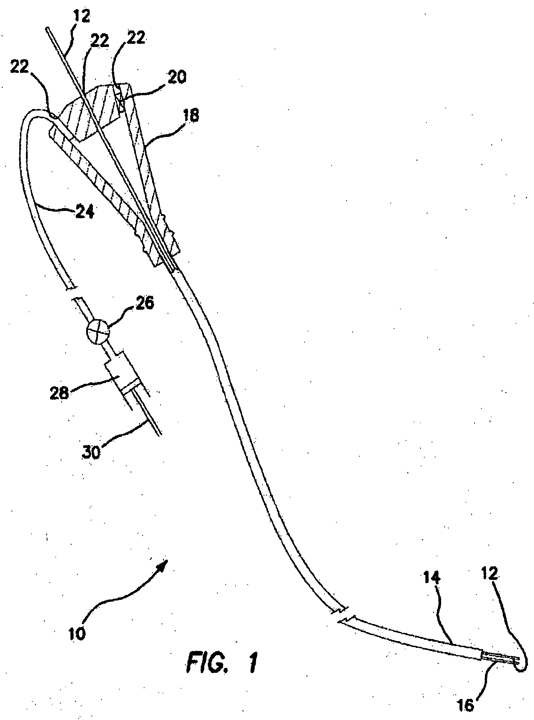

Shah, U.S. Patent Application Publication 2008/0188802 is directed to a multi-lumen lay-flat tubing, catheter articles comprising same, and methods of manufacture thereof. The balloon catheter as seen in Figure 1 includes a multi-lumen tube formed of a polymeric material such as polyurethane that is formed by beginning with two superposed sheets followed by the welding of the sheets. The resulting structure is a main tubular body 12 that is secured to an inflatable balloon 28 wherein the main tubular body 12 may have additional tubular passages such as shown at numerals 16 and 18. The additional tubular passages 16 and 18 may communicate with the balloon 28, as well as the main feed 226 which may be coupled to a pressurized gas source so that the gas flow may pass into the interior volume of the balloon 28. The manufacturing process involves the utilization of two superposed sheets of a polymeric material such as polyurethane which is then followed by a welding process that seal portions of the sheets together in order to form passages and seal off other portions of the superposed sheets.

Shah, U.S. Patent 6,712,832, is directed to a low-pressure medical balloon and the method of making same, is also included wherein thin films of thermoplastic polymeric material are used to form a balloon where a welding process that involves radio-frequency welding is utilized.

Shah, U.S. Patent 5,833,915, is directed to a method of welding polyurethane thin film.

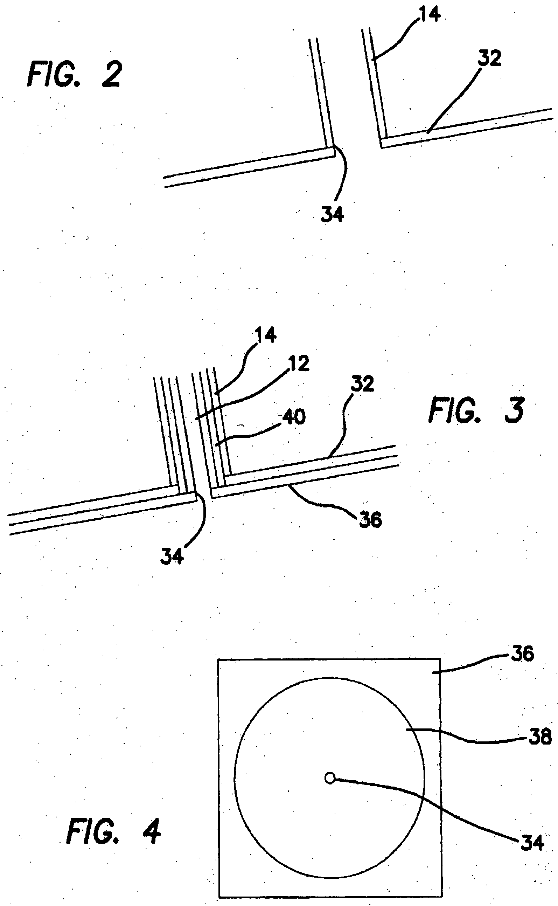

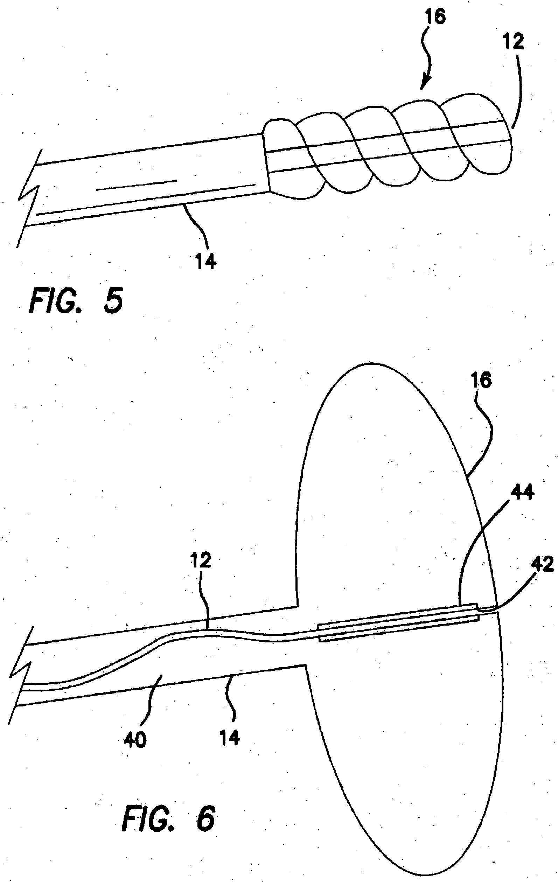

Koehn, U.S. Patent 3,050,066, is directed to retention catheters. Referring to the Figures, the catheter comprises an outer tube 16 and an inner tube 17 with an attached sleeve 18 made of a thin flexible material and sealed to the forward ends of the inner and outer tubes respectively. This is clearly seen in Figure 3. When the inner tube is extended from the outer tube as seen in Figure 5, the sleeve 18 may be inflated to form an annular enlargement 28 at the end of the catheter. Figure 4 shows the flexible sleeve 18 in a folded condition within outer tube 16.

Joergensen, U.S. Patent 7,022,106, is directed to a catheter having enhanced distal pushability. Referring to the Figures, the catheter 40 comprises an outer tube 42 and an inner tube 44 that extends coaxially within outer tube 42. A balloon 46 is attached to the distal end or inner tube 44. Balloon 46 is also attached proximally to outer tube 42 which may be inflated by means of lumen 43 which lies between outer tube 42 and inner tube 44. The tapered portion of outer tube 42 may include radiopaque marker bands 48.

Burgmeier, U.S. Patent Application Publication 2005/0215950 is also included for being directed to balloon catheter with a radiopaque portion. The catheter 10 is a structure that includes an inner tubular member 22 and an outer tubular member 26 that are coaxial with one another, and the balloon portion 28 is attached distally to the inner tube at numeral 32 and proximally to the outer tube 26 at numeral 30.

Dehdashtian, U.S. Patent 6,379,372, is directed to an endovascular delivery system. Referring to the Figures, the catheter assembly 10 includes an elongated catheter 130 which has a dual tube construction that includes an inner tube 142 and an outer tube 132. The inner tube 142 is slidably extensible distally and retractable proximally relative to the outer tube 132. A balloon 156 is attached at its proximal end 160 to outer tube 132 and its distal end 158 of balloon 156 is attached to sleeve 150, hence the inner tube 142, and thus the extension of inner tube 142 relative to the outer tube 132 facilitates the longitudinal stretching of the balloon 156.

Neary, U.S. Patent Application Publication 2005/0075711, is directed to a balloon catheter with selectable diameter and expandable length, is also included and comprises a coaxial bi-lumen design. A catheter body 210 and an internal inflation lumen 226 with attached inflatable balloon 220 gives rise to a variable expansion balloon catheter in order to provide a preselected diameter and length for the balloon.

Hijlkema, U.S. Patent 5,792,415, is directed to a method for manufacturing a balloon catheter. Hijlkema shows a balloon that may be folded into a small diameter. Referring to Figure 7, the balloon is seen in its deflated configuration for introduction into the introducer sheath 26 which has a relatively small inside diameter, and thus the balloon member 9 may be folded into the small diameter in order to fit within the sheath 26.

Kilpatrick, U.S. Patent 6,972,024, is directed to a method of treating vulnerable plaque, and Chernomorsky, U.S. Patent 6,302,839, is directed to a device and method for radiation therapy. Both include a provision for inflating a balloon catheter that has a pressure release valve in order to limit the inflation pressure for the volume of inflation in the balloon portion.

### Brief Summary of the Invention

A catheter balloon of the illustrated embodiment is characterized by a collar or pillow-shape giving it a high aspect ratio and may be made of any type of material and made by any type of process, although welding processes are preferred. The material may be extensible or nonextensible (compliant/noncompliant). Any material may be theoretically used for the balloon, although urethane and isoprene are preferred. The butt welding of the balloon material to the butt-ends of the inner and outer catheters allows for an unexpected flatness or closeness in wrapping the balloon material around an inner catheter so that the outer diameter of the outer catheter is closely approximated by the outer envelope of the wrapped balloon. Two flat squares of polyurethane are butt solvent-welded to pliable catheters and a hole punch out through the hollow catheter. The butt welding allows a much smaller diameter to be realized of the folded balloon on the catheter than is realizable through lap welding used in the prior art. The two hollow catheters are telescoped together with their squares and then the two squares are a circularly heat welded together. The excess material outside the circular weld is trimmed away. The thickness of the circular heat weld is easily and precisely controllable. The manufacturing method is substantially less costly than the prior art method of blow molding extensible biomedical occlusion balloons. The resulting manufactured balloon can be readily made with a high diameter to length aspect ratio and be attached to small catheters 6F and below, which was not a prior art capability.

The collapsed balloon is wrapped or folded around the inner catheter extended out of the inner catheter. An air syringe is then used to inflate the balloon which assumes the disk-pillow shape so that it can be employed in the coronary sinus system for occlusion near vein branches without blocking the adjacent branch. The high-diameter-to-axial-length ratio of the balloon, and a high-diameter-of-the-balloon-to-catheter diameter ratio, both of which are significant for small diameter catheters. This allows a larger diameter balloon to be realized at a smaller inflation volume for coronary sinus vessels up to 2.0 cm in diameter while not requiring additional amounts of balloon material, which would be the case with latex balloons on fixed attachments to the catheter, which inflate to spherical or long length shapes. This makes it easier to withdraw the balloon catheter into a small diameter delivery catheter or introducer.

The inner catheter is pulled inward or compressed into the outer catheter, but stays substantially on center on the inner catheter to stabilize and keep the balloon centered when and as it is inflated. The balloon has a selective stiffener or a short section of thin heat-shrink tubing on the distal portion of the inner catheter that enables the appropriate amount of axial stiffness to prevent balloon deflection, while maintaining a soft tip and flexible inner catheter. It is important that the inner catheter be as flexible as possible to avoid vessel trauma, but the distal portion extending through the balloon needs to be stiffened in order to achieve the shortening or pistoning action described as follows. Softness is also needed to allow the inner catheter to piston as described below. The inner catheter is hollow and is used to inject fluoroscopic dyes. Hence, the distal end of the inner catheter is kept free in the vessel and not occluded or blocked by being bent and having its orifice abutted against the vessel wall and hence must be stiff to avoid buckling.

The inner catheter accordions inside the outer catheter along the entire length inside the outer catheter to allow this "pistoning" of the inner catheter as its extended portion is shortened when the balloon inflates. The proximal ends of both catheters are fixed. The diameter of the balloon and hence the amount of extended inner catheter will vary on each application depending on vein diameter and the design automatically adjusts the two catheters accordingly. This ability to shorten is necessary to obtain the high aspect ratio of the balloon and the ability to have different amounts of shortening is necessary to have such a high aspect ratio balloon of differently adjusted diameters. The only way different diameter balloons of the prior art can be provided is by providing different lengths and different starting diameters of an expanding balloons. The prior art latex balloon is attached to a fixed length of the catheter and inflates in a generally spherical shape so that a greater diameter necessitates a greater length of the catheter to be provided as the balloon attachment base. This in turn requires a larger balloon making the balloon more difficult to withdraw into a small diameter delivery catheter (not shown) and is more prone due to the length to occlude branch coronary sinus vessels near the coronary sinus ostium. This limitation is not current in the self-adjusting high aspect ratio pillow balloon, which pulls or pushes the inner catheter into the outer catheter by amounts varying on the degree of the radial expansion of the balloon, always keeping the attachment base on the inner catheter to a length appropriate for the oblateness of the balloon depending on its degree of inflation.

More generally, the illustrated embodiments of the invention can be characterized in several alternative ways, which are summarized as follows. The illustrated embodiment is a balloon catheter for use with an introducer having an inner diameter comprising an outer catheter, a resilient inner catheter telescopically disposed in the outer catheter and extending distally therefrom, an annular space defined between the inner and outer catheters; and a high-aspect, distal, inflatable balloon having one end of the balloon coupled to the inner catheter and an opposing end of the balloon coupled to the outer catheter, the balloon having an interior fluidicly communicating with the annular space between the inner and outer catheters, the balloon being joined at its corresponding ends to the corresponding inner and outer catheters, so that the balloon when inflated buckles the inner catheter inside the outer catheter, is characterized by an outer diameter approximately 8 times the introducer inner diameter, a balloon diameter to length aspect ratio equal to or greater than 1, and a self adjusting inner catheter that automatically folds the balloon about the inner catheter as it distally extends from the outer catheter when the balloon is deflated so that the outer and inner catheters with the deflated balloon fit and is disposable through the introducer.

The illustrated embodiment is a coronary sinus balloon catheter for use with an introducer having an inner diameter of 6.5 French or less comprising an outer catheter, a resilient inner catheter telescopically disposed in the outer catheter and extending distally therefrom, an annular space defined between the inner and outer catheters, and a high-aspect, distal, inflatable balloon having one end of the balloon coupled to the inner catheter and an opposing end of the balloon coupled to the outer catheter, the balloon having an interior fluidicly communicating with the annular space between the inner and outer catheters, the balloon being joined at its corresponding ends to the corresponding inner and outer catheters, so that the balloon when inflated buckles the inner catheter inside the outer catheter, is characterized by a shape and size having an outer diameter equal to or greater than 1.5cm, a balloon diameter to length aspect ratio equal to or greater than 1, and a self adjusting inner catheter that automatically folds the balloon about the inner catheter as it distally extends from the outer catheter when the balloon is deflated so that the outer and inner catheters with the deflated balloon fit and is disposable through the introducer having an inner diameter of 6.5 French or less.

The illustrated embodiment is a balloon catheter comprising an outer catheter, a resilient inner catheter telescopically disposed in the outer catheter and extending distally therefrom, and a high-aspect, distal, inflatable balloon having one end of the balloon coupled to the inner catheter and an opposing end of the balloon coupled to the outer catheter, so that the balloon when inflated buckles the inner catheter inside the outer catheter, is characterized by a shape and size having an outer diameter equal to or greater than 1.5cm, a length less than the diameter, and a self adjusting inner catheter that automatically folds the balloon about the inner catheter as it distally extends from the outer catheter when the balloon is deflated.

The illustrated embodiment is a balloon catheter comprising an outer catheter, a resilient inner catheter telescopically disposed in the outer catheter and extending distally therefrom, and a high-aspect, distal, inflatable balloon having one end of the balloon coupled to the inner catheter and an opposing end of the balloon coupled to the outer catheter, so that the balloon when inflated rotates the inner catheter inside the outer catheter, is characterized by a shape and size having an outer diameter equal to or greater than 1.5cm, a length less than the diameter, and a self adjusting inner catheter that automatically rotationally folds the balloon about the inner catheter as it distally extends from the outer catheter when the balloon is deflated.

The illustrated embodiment is a balloon catheter for use with an introducer having an inner diameter comprising an outer elongate means for delivery through the introducer, a telescopic resilient inner elongate means telescopically disposed in the outer means and extending distally therefrom, an annular space defined between the inner and outer elongate means, and a high-aspect, distal, inflatable balloon means coupled between the inner and outer elongate means, an inflation means for communicating with the balloon means, so that the balloon means may be inflated with a fluid communicated the inflation means and when inflated is characterized by a shape and size having an outer diameter equal to or greater than 1.5cm, a diameter-to-length aspect ratio equal to or greater than 1, where the inner elongate means automatically folds the balloon means when the inner elongate means distally is extended from the outer elongate means when the balloon means is deflated, where the balloon means when deflated is disposable through the introducer having an inner diameter of 6.5 French or less, and where the balloon means is inflated the inner elongate means shortens by buckling inside the outer elongate means. The illustrated embodiment is a balloon catheter comprising an outer elongate means, a telescopic resilient inner elongate means telescopically disposed in the outer elongate means and extending distally therefrom, a high-aspect, distal, and an inflatable balloon means coupled between the outer and inner elongate means; means for selectively inflating the balloon means; so that the balloon means when inflated it is characterized by a shape and size having an outer diameter equal to or greater than 1.5cm a length less than the diameter, and where the inner elongate means is automatically self-adjusting to fold the balloon means about the inner elongate means when the inner elongate means is distally extended from the outer elongate means and when the balloon is deflated, and where the balloon is inflated the inner elongate means is automatically self-shortening.

The illustrated embodiment is a method of using a balloon catheter with an introducer having an inner diameter of 6.5 French or less comprising the steps of providing a high-aspect, distal, inflatable balloon having one end of a balloon coupled to an inner catheter and an opposing end of the balloon coupled to an outer catheter, the inner catheter being telescopically disposed within the outer catheter, and folding the balloon when deflated flatly around a portion of the inner catheter distally extending from the outer catheter so that the inner and outer catheters with the folded deflated balloon is capable of being telescopically disposed through the introducer.

The method further comprises the steps of telescopically disposing the folded deflated balloon and inner and outer catheters through the introducer, manipulating the introducer into a proximity of an anatomical site of application, extending the folded deflated balloon and inner and outer catheters distally from the introducer to the anatomical site, and inflating the balloon at the anatomical site of application until the balloon has a shape characterized an outer diameter equal to or greater than 1.5cm, a balloon diameter to length aspect ratio equal to or greater than 1 or less as may be restricted by the anatomical site of application, inflation of the balloon simultaneously automatically adjusting the inner catheter length by buckling the inner catheter inside the outer catheter.

The method further comprises the step of deflating the balloon and simultaneously automatically adjusting the inner catheter length by unbuckling the inner catheter inside the outer catheter so that the balloon automatically flatly folds around the inner catheter, so that the balloon, inner catheter and outer catheter can be telescopically withdrawn into the introducer.

The illustrated embodiment is a method of using balloon catheter in combination with an introducer comprising the steps of providing an outer catheter, providing a telescopic resilient inner catheter telescopically disposed in the outer catheter and extending distally therefrom, providing a high-aspect, distal, inflatable balloon having one end of the balloon coupled to the inner catheter and an opposing end of the balloon coupled to the outer catheter and flatly folded around a distal portion of the inner catheter, telescoping disposing the balloon, and inner and outer catheter within the introducer, delivering the balloon, and inner and outer catheter using the introducer to an application site ready for placement of the balloon in a patient, inflating the balloon so that the balloon when inflated is characterized by being capable of assuming a shape and size having an outer diameter equal to or greater than 1.5cm a length less than the diameter or less as may be restricted by the application site, simultaneously shortening the inner catheter by buckling it within the outer catheter; subsequently deflating the balloon and automatically folding the balloon about the inner catheter as it distally extends from the outer catheter; and simultaneously lengthening the inner catheter by unbuckling it within the outer catheter.

The illustrated embodiment is a balloon catheter comprising an outer catheter, an inner catheter telescopically disposed in the outer catheter and extending distally therefrom, a high-aspect, distal, inflatable balloon having one end of the balloon coupled to the inner catheter and an opposing end of the balloon coupled to the outer catheter, the balloon being wrapped onto the inner catheter so that when inflated the balloon rotates and/or pulls the inner catheter inside the outer catheter, and means for storing rotational and/or axial energy when the balloon is inflated and releasing the stored energy when the balloon is deflated to collapse and to closely wrap the balloon back onto the inner catheter.

The inner catheter is resilient and the means comprises buckling of the resilient inner catheter, the means comprises torsion of the resilient inner catheter, the means comprises a separate compression or torsion spring coupled to the inner catheter or outer catheter, the means comprises an air piston coupled to the inner or outer catheter, or the means comprises a mechanical, pneumatic, hydraulic or electrical device for reciprocating axial and/or rotational relative movement between to the inner and outer catheters.

The balloon catheter further comprises a distal stiffener coupled to the distal portion of the inner catheter to reduce deflection of the inner catheter when the balloon is inflated.

The balloon catheter further comprises radiopaque markers disposed on or near a proximal or distal end of the balloon.

The balloon catheter further comprises a source of fluidic pressure communicated to the balloon for inflating balloon and a pressure relief valve communicating with the balloon for automatically limiting fludic pressure supplied to the balloon to a level below that which would rupture the balloon.

The balloon catheter further comprises a hub for defining a common manifold intercommunicating fluidic pressure among the source of fluidic pressure, the pressure relief valve, and an axial coaxial lumen defined between the outer and inner catheters and communicating with the balloon.

While the apparatus and method has or will be described for the sake of grammatical fluidity with functional explanations, it is to be expressly understood that the claims, unless expressly formulated under 35 USC 112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 USC 112 are to be accorded full statutory equivalents under 35 USC 112. The invention can be better visualized by turning now to the following drawings wherein like elements are referenced by like numerals.

### Brief Description of the Drawings

Fig. 1 is a partial cutaway side view of the catheter system of the illustrated embodiment of the invention.
Fig. 2 is a longitudinal side cross-sectional view of the distal end of the outer catheter and a portion of the balloon as seen in a first step of fabrication of the balloon where the outer catheter is solvent butt welded to a proximal half of the balloon.
Fig. 3 is a longitudinal side cross-sectional view of the distal end of the outer catheter and a portion of the balloon as seen in a second step of fabrication of the balloon where the inner catheter is solvent butt welded to a distal half of the balloon.
Fig. 4 is a plan distal view of the distal end of the inner catheter and a portion of the balloon as seen in a third step of fabrication of the balloon where a circular weld is formed and the collar or pillow shape of the balloon formed.
Fig. 5 is a side plan view of the distal end of the catheter showing the inner catheter distally extended from the outer catheter and the balloon twisted or wrapped around the inner catheter.
Fig. 6 is a side plan view of the distal end of the catheter showing the balloon inflated with the inner catheter being pulled into and buckled and twisted into the outer catheter by the inflation of the balloon.

The invention and its various embodiments can now be better understood by turning to the following detailed description of the preferred embodiments which are presented as illustrated examples of the invention defined in the claims. It is expressly understood that the invention as defined by the claims may be broader than the illustrated embodiments described below.

### Detailed Description of the Preferred Embodiments

The balloon catheter 10 of the illustrated embodiment is a single use medical device designed to provide temporary vascular occlusion or balloon catheter flotation while providing a port for distal contrast injection or guidewire placement. The catheter 10 may be used for procedures requiring angiography, wedge pressure measurements, or any other procedure where a vessel needs to be occluded while maintaining a distal lumen. The balloon catheter 10 in its deflated state passes through a small introducer, and in its inflated state increases to a size 8-10 times the diameter of the introducer ID.

The balloon catheter 10 that can fit through or be telescopically disposed in an introducer (not shown), such as a cardiac sinus introducer. The balloon catheter 10 as shown in partial longitudinal cross-sectional view in Fig. 1 is comprised of a telescoping inner catheter 12 and outer catheter 14 with a folded or wrapped balloon 16 that is attached to the inner catheter 12 and outer catheter 14. The inner and outer catheters 12, 14 are fixed at or near their proximal ends into a hub 18. The hub 18 is provided with at least intercommunicating proximal ports 22. A pressure relief valve 20 is disposed into one of the ports 22, inner catheter 12 is sealed in and extends through a second one of the ports 22 and an air or fluid supply line 24 is sealed or coupled to the third one of the ports 22. Line 24 is coupled to a control valve 26 to which an air or fluid syringe 28 is coupled. In the illustrated embodiment syringe 28 is operated with a manual piston 30, but any conventional source of low pressure air or fluid may be substituted for syringe 28 and piston 30.

The balloon 16 is assembled as usual with the distal end of the balloon bonded to the inner catheter 12 and the proximal end of the balloon 16 bonded to the outer catheter 14. When the proximal ends of the inner and outer catheter 12, 14 are being assembled into the molded manifold hub 18, the inner catheter 12 may be rotated with respect to the outer catheter 14 until the balloon 16 is completely wrapped, but not stressed or stretched. Then the proximal ends of both the inner and outer catheters 12, 14 are bonded to the manifold hub 18 and secured.

An annular, coaxial gap is provided between the length of inner catheter 12 and outer catheter 14 along their parallel lengths into which air from syringe 28 is communicated to the interior of balloon 16. Turn to Fig. 2 where the fabrication of balloon 16 is depicted. The balloon 16 is manufactured using a welding process instead of a blowing process so that it can have a more radially extending shape from the catheters 12 and 14 and is fabricated at a significantly lower cost. The balloon material is semicompliant, though both compliant and non-compliant balloon materials can also be used. In the illustrated embodiment the balloon material and catheters 12 and 14 are made of polyurethane, enabling their assembly to be solvent bonded creating a chemical bond. The longitudinal cross-sectional view of Fig. 2 shows that outer catheter has it distal butt ends flushly disposed against a flat square of balloon material 32 and is solvent welded thereto. A central hole 34 is defined through balloon material into the lumen of outer catheter 14. As shown in the longitudinal cross-sectional view of Fig. 3 a matching square of balloon material 36 is laid next to the square of balloon material 32 and inner catheter 12 similarly butt solvent welded to the square of balloon material 36 with hole 34 also being extend between the lumen of inner catheter 12 and square of balloon material 36. As shown in the plan view of Fig. 4 the squares of balloon material 32 and 36 are then heat welded in a circular or other weld line 38 with the material exterior to the weld line 38 cutaway leaving in the case of the illustrated embodiment a flat, deflated circular disc. It is clear that if shapes other than circular are desired, weld line 38 can be appropriated shaped, e.g. into ellipses or freely contoured shapes.

As diagrammatically depicted in the side plan view of Fig, 5, inner catheter 12 is then extended distally from outer catheter 14 to extend balloon 16 flatly along the length of inner catheter 12. At the same time inner catheter 12 is rotated to twist or wrap balloon 16 around inner catheter 12 as it is distally extended, thereby closely folding or wrapping balloon 16 around the length of inner catheter 12. Alternatively, inner catheter 12 can be extended without rotation so that any slack in the balloon 16 is elongated. Then when the balloon 16 is inflated, it pulls the inner catheter 12 inside the outer catheter 14. As described above in connection with the fabrication steps of the balloon 16, it is to be noted that it is attached to the face or butt end of each the inner and outer catheters 12, 14 to enable balloon 16 to maintain a low profile for insertion and withdrawal from a small cardiac sinus introducer (not shown). The twisting or wrapping of balloon 16 around inner catheter 12 also assists in its close folding around inner catheter 12 thereby allowing it to assume an effect deflated outer diameter approximately equal to that of outer catheter 14 or at least to easily fit within the inner diameter of the introducer through which outer catheter 14 will later be telescopically disposed. In extending and twisting balloon 16 onto the distal length of inner catheter 12 extended distally from outer catheter 14, balloon 16 is not stretched to any substantial extent. The material of balloon 16 is chosen so that it is pliant to deformation and twisting prior to being stretched without creating a high resisting or restorative elastic or resilient force. As a result, when balloon 16 is configured in the extended and twisted or wrapped configuration of Fig. 5 there is no or very little torsion or compression on inner catheter 12 from balloon 16 tending to either pull inner catheter into outer catheter 14 or to rotate inner catheter 12 to any appreciable extent. The elements of the entire system are relaxed and balloon 16 is able to easily return to its original untwisted configuration, if so driven by a force, without substantial resistance from the material of balloon 16 even after a long shelf life in the twisted configuration of Fig. 5. Once configured as shown in Fig. 5, inner and outer catheters 12, 14 are then fixed into position by glue or other means into hub 18. Balloon 16 thus stays in its wrapped configuration until driven by a force into a different configuration by inflation.

Fig. 6 is a diagrammatic side cross-sectional view of balloon 16 after it has been inflated. Air is supplied from syringe 18 into the coaxial space 40 between catheters 12 and 14. As balloon 16 is inflated it is forced away from inner catheter 12 and will both pull inner catheter 12 into outer catheter 14 as well as simultaneously rotating it at the same time. Since inner catheter 12 is fixed both longitudinally and rotationally with respect to outer catheter 14 at its proximal end by both being fixed to hub 18, the inflation of balloon 16 causes inner catheter 12 to twist like a torsional spring thereby storing rotational energy into the catheter 12 and causes it to longitudinal buckle as shown in greatly exaggerated form in Fig. 6 along its length storing translational energy into catheter 12.

When the balloon 16 is inflated, as described below it unwraps and the balloon inflation causes axial and/or rotational motion of the inner catheter 12 relative to the outer catheter 14. The energy of inflation is stored, so when the balloon 16 is deflated, the movement is reversed causing the balloon 16 to resume its original folded or wrapped shape as driven by the energy stored in catheter 12 to resume the wrapped configuration of Fig. 5. It is possible to store the inflation-driven movement (kinetic energy) into converted potential energy by many different means, however in the preferred embodiment the means used is primarily the resilient nature of the inner catheter 12. For example, in addition to the torsional or compression of inner catheter 12, a separate compression or torsion spring may be provided in catheter 10, an air piston, or any other known mechanical, pneumatic, hydraulic or electrical device for reciprocating the axial and/or rotational movement relative to the inner and outer catheters 12, 14.

Unlike prior art balloons which exhibited an increased reluctance or resistance to folding flatly after inflating the higher the aspect ratio of the balloon (i.e. balloon radial diameter to longitudinal length), the balloon catheter 10 of the illustrated embodiment prefers to be closely wrapped or folded and is driven by the spring action of catheter 12, which can be chosen to have a longitudinal and/or torsional resiliency of any magnitude necessary to obtain a close or tight rewrapping of balloon 16 upon deflation. This in turn allows balloon catheter 10 to be easily withdrawn through very small ID introducers nearly without regard to the magnitude of the aspect ratio of the balloon when inflated.

It can now be appreciated that the balloon radial diameter in a direction perpendicular to the catheters' longitudinal axes is larger than a typical vascular occlusion balloons allowing it to occlude vessels up to 1.75cm (though any size balloon is conceived). The balloon and catheter design of the illustrated embodiment permit the catheter 10 to be inserted and removed from a small diameter introducer system (6F for the illustrated embodiment). The balloon 16 is mounted on a "floating" coaxial (telescoping) catheter 12, 14 enabling the inner and outer catheters 12, 14 to automatically adjust to the balloon shape as it is inflated and deflated, i.e. the higher the inflation and higher the aspect ratio achieved, inner catheter 12 is pulled inwardly into catheter 14 and rotates by automatically and exactly by the right amount to accommodate the degree of inflation or unwrapping. The automatic telescoping, self adjusting catheter design allows the balloon to fold and fit through a small inner diameter delivery catheter while allowing the balloon to inflate to its maximum diameter with the minimum length, thereby enabling a smaller volume which is desirable in the event of balloon rupture. In other words, it is a property of an oblate or high aspect ratio balloon to decrease volume as the degree of its aspect ratio increases with increasing inflation.

The balloon inflation port 22 coupled to syringe 28 is in fluid communication with a fixed pressure relief valve 20 in hub 18 to regulate the pressure to the balloon 16 enabling precise occlusion pressure while preventing vessel trauma or dissection. If the surgeon pushes on piston 30 too much or too fast creating an overpressure which might tend to lead to rupture of balloon 16, pressure relief valve 20 automatically opens at a pressure well below the rupture limit of balloon 16 thereby rendering rupture impossible and making the use of catheter 10 fail-safe.

The catheter injection lumen 42 in inner catheter 12 is also sized to allow a .025" diameter guidewire to be placed (though smaller or larger lumens are also conceived), thereby increasing the utility and guidability of catheter 10 into cardiac sinus applications.

In one embodiment balloon 16 has a selective stiffener 44 shown in Fig. 6 added to the distal portion of inner catheter 12 to enable the appropriate amount of axial stiffness of catheter 12 to prevent undesired deflection in the vessel when the balloon is inflated, while maintaining a soft tip of and a flexible overall length of catheter 12. Stiffener 44 is comprised of a short length of shrink-wrap tubing to provide radial stiffness to the distal portion of catheter 12.

In another embodiment the balloon 16 has radiopaque markers or coils (not shown) added to its surface or embedded into the balloon material, or disposed on the outer and inner catheter at the proximal and distal ends of the balloon for exact balloon placement under x-ray.

Therefore, it must be understood that the illustrated embodiment has been set forth only for the purposes of example and that it should not be taken as limiting the invention as defined by the following claims. For example, notwithstanding the fact that the elements of a claim are set forth below in a certain combination, it must be expressly understood that the invention includes other combinations of fewer, more or different elements, which are disclosed in above even when not initially claimed in such combinations. A teaching that two elements are combined in a claimed combination is further to be understood as also allowing for a claimed combination in which the two elements are not combined with each other, but may be used alone or combined in other combinations. The excision of any disclosed element of the invention is explicitly contemplated as within the scope of the invention.

The words used in this specification to describe the invention and its various embodiments are to be understood not only in the sense of their commonly defined meanings, but to include by special definition in this specification structure, material or acts beyond the scope of the commonly defined meanings. Thus if an element can be understood in the context of this specification as including more than one meaning, then its use in a claim must be understood as being generic to all possible meanings supported by the specification and by the word itself.

The definitions of the words or elements of the following claims are, therefore, defined in this specification to include not only the combination of elements which are literally set forth, but all equivalent structure, material or acts for performing substantially the same function in substantially the same way to obtain substantially the same result. In this sense it is therefore contemplated that an equivalent substitution of two or more elements may be made for any one of the elements in the claims below or that a single element may be substituted for two or more elements in a claim. Although elements may be described above as acting in certain combinations and even initially claimed as such, it is to be expressly understood that one or more elements from a claimed combination can in some cases be excised from the combination and that the claimed combination may be directed to a subcombination or variation of a subcombination.

Insubstantial changes from the claimed subject matter as viewed by a person with ordinary skill in the art, now known or later devised, are expressly contemplated as being equivalently within the scope of the claims. Therefore, obvious substitutions now or later known to one with ordinary skill in the art are defined to be within the scope of the defined elements.

The claims are thus to be understood to include what is specifically illustrated and described above, what is conceptionally equivalent, what can be obviously substituted and also what essentially incorporates the essential idea of the invention.

## Claims

1. A balloon catheter for use with an introducer having an inner diameter comprising:
an outer catheter;
a resilient inner catheter telescopically disposed in the outer catheter and extending distally therefrom,
an annular space defined between the inner and outer catheters; and
a distal, inflatable balloon having one end of the balloon coupled to the inner catheter and an opposing end of the balloon coupled to the outer catheter,
the balloon having an interior fluidicly communicating with the annular space between the inner and outer catheters,
the inner catheter being automatically adjustable to the balloon shape on inflation and deflation,
the balloon being attached at its corresponding ends to both of the corresponding inner and outer catheters, so that on inflation of the balloon
the inner catheter is bucklable inside the outer catheter,
the balloon having a balloon diameter-to-length aspect ratio being equal to or greater than 1,
wherein the self-adjusting inner catheter is configured to automatically fold the balloon about the inner catheter on distal extension of the inner catheter from the outer catheter when the balloon is deflated, so that the outer and inner catheters and the deflated balloon fit through and are disposable through the introducer, and further
where the balloon is wrapped onto the inner catheter so that when inflated the balloon rotates and/or pulls the inner catheter inside the outer catheter thereby buckling the proximal portion of the inner catheter within the outer catheter; and
the buckling of the proximal portion of the inner catheter within the outer catheter providing a means for storing rotational and/or axial energy when the balloon is inflated and releasing the stored energy when the balloon is deflated to collapse and to closely wrap the balloon back onto the inner catheter.

2. The balloon catheter according to claim 1 for use with an introducer having an inner diameter of 2.17mm or less.

3. The balloon catheter according to claims 1 or 2 where the balloon has an outer diameter equal to or greater than 1.5cm when inflated.

4. The balloon catheter of any of the preceding claims where the means for storing rotational and/or axial energy when the balloon is inflated and releasing the stored energy when the balloon is deflated, comprises torsion of the resilient inner catheter.

5. The balloon catheter of any of the claims 1-3 where the means for storing rotational and/or axial energy when the balloon is inflated and releasing the stored energy when the balloon is deflated comprises a separate compression or torsion spring coupled to the inner catheter or outer catheter.

6. The balloon catheter of any of the claims 1-3 where the means for storing rotational and/or axial energy when the balloon is inflated and releasing the stored energy when the balloon is deflated comprises an air piston coupled to the inner or outer catheter.

7. The balloon catheter of any of the claims 1-3 where the means for storing rotational and/or axial energy when the balloon is inflated and releasing the stored energy when the balloon is deflated comprises a mechanical, pneumatic, hydraulic or electrical device for reciprocating axial and/or rotational relative movement between to the inner and outer catheters.

8. The balloon catheter according to any of the preceding claims further comprising a distal stiffener coupled to the distal portion of the inner catheter to reduce deflection of the inner catheter when the balloon is inflated.

9. The balloon catheter according to any of the preceding claims further comprising radiopaque markers disposed on or near a proximal or distal end of the balloon.

10. The balloon catheter according to any of the preceding claims further comprising a pressure relief valve communicating with the balloon for automatically limiting fluidic pressure supplied to the balloon to a level below that which would rupture the balloon.

11. The balloon catheter of claim 10 further comprising a hub for defining a common manifold intercommunicating fluidic pressure among the source of fluidic pressure, the pressure relief valve, and an axial coaxial lumen defined between the outer and inner catheters and communicating with the balloon.

12. The balloon catheter according to any of the preceding claims being a coronary sinus catheter.

13. A system comprising a balloon catheter according to any of the preceding claims and an introducer for the balloon catheter, wherein the balloon catheter has an outer diameter approximately 8 times larger than the introducer inner diameter when inflated.

## Patentansprüche

1. Ballonkatheter zur Verwendung mit einer Einführeinrichtung, die einen Innendurchmesser aufweist, umfassend:
einen äußeren Katheter,
einen elastischen inneren Katheter, der teleskopisch in dem äußeren Katheter angeordnet ist und der sich von diesem distal erstreckt,
einen zwischen den inneren und äußeren Kathetern festgelegten ringförmigen Raum und einen distalen aufblasbaren Ballon, dessen eines Ballonende mit dem inneren Katheter gekoppelt ist und dessen gegenüberliegendes Ballonende mit dem äußeren Katheter gekoppelt ist,
wobei der Ballon einen Innenraum aufweist, der fluidmäßig mit dem ringförmigen Raum zwischen den inneren und äußeren Kathetern in Verbindung ist,
wobei der innere Katheter automatisch an die Ballonform beim Aufblasen und Entleeren anpassbar ist,
wobei der Ballon an seinen entsprechenden Enden an den beiden entsprechenden inneren und äußeren Kathetern derart angebracht ist, dass beim Aufblasen des Ballons der innere Katheter innerhalb des äußeren Katheters aufwölbbar ist,
wobei der Ballon ein Ballondurchmesser-zu-Ballonlänge-Streckenverhältnis aufweist, welches gleich oder größer als 1 ist,
wobei der sich selbst anpassende innere Katheter so gestaltet ist, um den Ballon, wenn dieser entleert wird, automatisch um den inneren Katheter an dessen distaler Erstreckung aus dem äußeren Katheter zu falten, derart, dass der äußere Katheter, der innere Katheter und der entleerte Ballon durch die Einführeinrichtung passen und durch diese leitbar sind,
wobei ferner in dem Fall, dass der Ballon auf den inneren Katheter gewickelt ist, er dann, wenn er aufgeblasen wird, den inneren Katheter innerhalb des äußeren Katheters dreht und/oder zieht und dadurch den proximalen Teil des inneren Katheters innerhalb des äußeren Katheters aufwölbt
und wobei das Aufwölben des proximalen Teiles des inneren Katheters innerhalb des äußeren Katheters ein Mittel zur Speicherung von Rotations- und/oder Axialenergie, wenn der Ballon aufgeblasen wird, und zur Freigabe der gespeicherten Energie bereitstellt, wenn der Ballon entleert wird, um den Ballon zusammenfallen zu lassen und eng auf den inneren Katheter zurückzuwickeln.

2. Ballonkatheter nach Anspruch 1 zur Verwendung mit einer Einführeinrichtung, die einen Innendurchmesser von 2,17mm oder weniger aufweist.

3. Ballonkatheter nach Anspruch 1 oder 2, wobei der Ballon einen Außendurchmesser aufweist, der gleich oder größer als 1,5cm ist, wenn er aufgeblasen ist.

4. Ballonkatheter nach einem der vorhergehenden Ansprüche, wobei das Mittel zur Speicherung von Rotations- und/oder Axialenergie, wenn der Ballon aufgeblasen wird, und zur Freigabe der gespeicherten Energie, wenn der Ballon entleert wird, die Torsion des elastischen inneren Katheters umfasst.

5. Ballonkatheter nach einem der Ansprüche 1 bis 3, wobei das Mittel zur Speicherung von Rotations- und/oder Axialenergie, wenn der Ballon aufgeblasen wird, und zur Freigabe der gespeicherten Energie, wenn der Ballon entleert wird, eine mit dem inneren Katheter oder dem äußeren Katheter gekoppelte gesonderte Druck- oder Torsionsfeder umfasst.

6. Ballonkatheter nach einem der Ansprüche 1 bis 3, wobei das Mittel zur Speicherung von Rotations- und/oder Axialenergie, wenn der Ballon aufgeblasen wird, und zur Freigabe der gespeicherten Energie, wenn der Ballon entleert wird, einen mit dem inneren oder äußeren Katheter gekoppelten Luftkolben umfasst.

7. Ballonkatheter nach einem der Ansprüche 1 bis 3, wobei das Mittel zur Speicherung von Rotations- und/oder Axialenergie, wenn der Ballon aufgeblasen wird, und zur Freigabe der gespeicherten Energie, wenn der Ballon entleert wird, eine mechanische, pneumatische, hydraulische oder elektrische Einrichtung für eine hin- und hergehende Axial- und/oder Rotations-Relativbewegung zwischen den inneren und äußeren Kathetern umfasst.

8. Ballonkatheter nach einem der vorhergehenden Ansprüche, ferner umfassend eine distale Versteifung, die mit dem distalen Teil des inneren Katheters gekoppelt ist, um ein Auslenken des inneren Katheters zu verringern, wenn der Ballon aufgeblasen wird.

9. Ballonkatheter nach einem der vorhergehenden Ansprüche, ferner umfassend an oder nahe eines proximalen oder distalen Endes des Ballons angeordnete strahlungsundurchlässige Marker.

10. Ballonkatheter nach einem der vorhergehenden Ansprüche, ferner umfassend ein Druckentlastungsventil, welches mit dem Ballon in Verbindung ist, um den dem Ballon zugeführten Fluiddruck automatisch auf einen Pegel zu begrenzen, der unterhalb desjenigen liegt, welcher den Ballon platzen ließe.

11. Ballonkatheter nach Anspruch 10, ferner umfassend eine Büchse zum Festlegen eines gemeinsamen Verteilers, durch den ein Fluiddruck zwischen der Fluiddruckquelle, dem Druckentlastungsventil und einem zwischen den äußeren und inneren Kathetern festgelegten Lumen zusammenhängt und der mit dem Ballon in Verbindung steht.

12. Ballonkatheter nach einem der vorhergehenden Ansprüche, wobei er ein Koronarsinus-Katheter ist.

13. Anlage, umfassend einen Ballonkatheter nach einem der vorhergehenden Ansprüche und eine Einführeinrichtung für den Ballonkatheter, der dann, wenn er aufgeblasen ist, einen Außendurchmesser aufweist, welcher acht Mal größer ist als der Innerdurchmesser der Einführeinrichtung.

## Revendications

1. Cathéter à ballonnet destiné à être utilisé avec un dispositif d'introduction présentant un diamètre interne comprenant :
un cathéter externe ;
un cathéter interne élastique, disposé de manière télescopique dans le cathéter externe et s'étendant de manière distale à partir de celui-ci,
un espace annulaire défini entre les cathéters interne et externe ; et
un ballonnet distal pouvant être gonflé, une première extrémité du ballonnet étant couplée au cathéter interne et une extrémité opposée du ballonnet étant couplée au cathéter externe,
le ballonnet présentant une partie interne en communication fluidique avec l'espace annulaire entre les cathéters interne et externe,
le cathéter interne pouvant être ajusté automatiquement à la forme du ballonnet lors du gonflage et du dégonflage,
le ballonnet étant fixé, au niveau de ses extrémités correspondantes, sur chacun des cathéters interne et externe correspondants, de telle sorte que, lors du gonflage du ballonnet :
le cathéter interne peut être formé en boucle à l'intérieur du cathéter externe,
le ballonnet présentant un rapport diamètre sur longueur du ballonnet qui est supérieur ou égal à 1,
dans lequel le cathéter interne pouvant être ajusté automatiquement est configuré de manière à plier automatiquement le ballonnet autour du cathéter interne lors de l'extension distale du cathéter interne à partir du cathéter externe lorsque le ballonnet est dégonflé, de telle sorte que les cathéters externe et interne et le ballonnet dégonflé passent à travers le dispositif d'introduction et peuvent être disposés par l'intermédiaire de celui-ci, et en outre
dans lequel le ballonnet est enroulé sur le cathéter interne de telle sorte que, lorsqu'il est gonflé, le ballonnet tourne et/ou entraîne le cathéter interne à l'intérieur du cathéter externe, formant ainsi en boucle la partie proximale du cathéter interne à l'intérieur du cathéter externe ; et
la formation en boucle de la partie proximale du cathéter interne à l'intérieur du cathéter externe constituant un moyen de stockage d'énergie de rotation et/ou axiale lorsque le ballonnet est gonflé, et de libération l'énergie stockée lorsque le ballonnet est dégonflé de manière à aplatir et à enrouler étroitement de nouveau le ballonnet sur le cathéter interne.

2. Cathéter à ballonnet selon la revendication 1, destiné à une utilisation avec un dispositif d'introduction présentant un diamètre interne inférieur ou égal à 2,17 mm.

3. Cathéter à ballonnet selon les revendications 1 ou 2, dans lequel le ballonnet présente un diamètre externe supérieur ou égal à 1,5 cm lorsqu'il est gonflé.

4. Cathéter à ballonnet selon l'une quelconque des revendications précédentes, dans lequel le moyen destiné à stocker l'énergie de rotation et/ou axiale lorsque le ballonnet est gonflé et à libérer l'énergie stockée lorsque le ballonnet est dégonflé, comprend la torsion du cathéter interne élastique.

5. Cathéter à ballonnet selon l'une quelconque des revendications 1 à 3, dans lequel le moyen destiné à stocker l'énergie de rotation et/ou axiale lorsque le ballonnet est gonflé et à libérer l'énergie stockée lorsque le ballonnet est dégonflé, comprend un ressort de compression ou de torsion distinct couplé au cathéter interne ou au cathéter externe.

6. Cathéter à ballonnet selon l'une quelconque des revendications 1 à 3, dans lequel le moyen destiné à stocker l'énergie de rotation et/ou axiale lorsque le ballonnet est gonflé et à libérer l'énergie stockée lorsque le ballonnet est dégonflé comprend un piston pneumatique couplé au cathéter interne ou externe.

7. Cathéter à ballonnet selon l'une quelconque des revendications 1 à 3, dans lequel le moyen destiné à stocker l'énergie de rotation et/ou axiale lorsque le ballonnet est gonflé et à libérer l'énergie stockée lorsque le ballonnet est dégonflé comprend un dispositif mécanique, pneumatique, hydraulique ou électrique afin d'assurer un mouvement relatif alternatif axial et/ou de rotation entre les cathéters interne et externe.

8. Cathéter à ballonnet selon l'une quelconque des revendications précédentes comprenant en outre un raidisseur distal couplé à la partie distale du cathéter interne afin de réduire la déformation du cathéter interne lorsque le ballonnet est gonflé.

9. Cathéter à ballonnet selon l'une quelconque des revendications précédentes comprenant en outre des repères radio-opaques disposés sur l'extrémité proximale ou distale du ballonnet ou à proximité de celle-ci.

10. Cathéter à ballonnet selon l'une quelconque des revendications précédentes comprenant en outre un clapet de limitation de pression communiquant avec le ballonnet afin de limiter automatiquement la pression fluidique délivrée au ballonnet à un niveau inférieur à celui qui pourrait rompre le ballonnet.

11. Cathéter à ballonnet selon la revendication 10, comprenant en outre un moyeu destiné à définir un collecteur commun assurant une communication de pression fluidique entre la source de pression fluidique, le clapet de surpression et une lumière axiale, coaxiale, définie entre les cathéters externe et interne et communiquant avec le ballonnet.

12. Cathéter à ballonnet selon l'une quelconque des revendications précédentes qui constitue un cathéter de sinus coronarien.

13. Dispositif comprenant un cathéter à ballonnet selon l'une quelconque des revendications précédentes et un dispositif d'introduction pour le cathéter à ballonnet, dans lequel le cathéter à ballonnet présente un diamètre externe approximativement 8 fois supérieur au diamètre interne du dispositif d'introduction lorsqu'il est gonflé.
